Europäisches Patentamt

European Patent Office

Office européen des brevets

(11) Publication number : **0 489 771 B1**

(12) **EUROPEAN PATENT SPECIFICATION**

(45) Date of publication of patent specification :
**11.05.94 Bulletin 94/19**

(51) Int. Cl.⁵ : **C07K 15/28, G01N 33/80, A61K 39/395**

(21) Application number : **90912594.0**

(22) Date of filing : **31.08.90**

(86) International application number :
**PCT/EP90/01463**

(87) International publication number :
**WO 91/03492 21.03.91 Gazette 91/07**

(54) **MODIFIED HUMAN ANTI-Rh(D) ANTIBODIES.**

(30) Priority : **01.09.89 GB 8919761**

(43) Date of publication of application :
**17.06.92 Bulletin 92/25**

(45) Publication of the grant of the patent :
**11.05.94 Bulletin 94/19**

(84) Designated Contracting States :
**AT BE CH DE DK ES FR GB IT LI LU NL SE**

(56) References cited :
**EP-A- 0 022 669
WO-A-89/02443
WO-A-89/02600
GB-A- 2 066 259
Proc. Natl. Acad. Sci., vol. 74, no. 6, June 1977, D.G. Romans et al., pp.2531-2535
Transfusion, vol. 27, 1987, E.C. Jones et al., pp. 142-144
Immunology, vol. 67, August 1989, A.G. Hadley et al., pp. 550-552**

(56) References cited :
**Immunology, vol. 62, Nov. 1987, E. Wiener et al., pp. 401-404
Proc. Natl. Acad. Sci. USA, vol. 81, May 1984, D. Bron et al., pp. 3214-3217
Immunology, vol. 58, 1986, K.M. Thompson et al., pp. 157-160**

(73) Proprietor : **National Blood Authority
Oak House, Reeds Crescent
Watford, Herts. WD1 1QH (GB)**

(72) Inventor : **SCOTT, Marion, Lesley
Flat 16 5 Royal York Crescent Clifton
Bristol BS8 4JW (GB)**
Inventor : **GUEST, Alan, Robert
5 Chequers Court Palmers Leaze
Bradley Stoke South Bristol BS12 0HD (GB)**
Inventor : **ANSTEE, David, John
1 Cavendish Road Henleaze
Bristol BS9 4DZ (GB)**

(74) Representative : **Holmes, Michael John et al
Frank B. Dehn & Co. European Patent
Attorneys Imperial House 15-19 Kingsway
London, WC2B 6UZ, (GB)**

Jouve, 18, rue Saint-Denis, 75001 PARIS

## Description

The present invention relates to reduced and S-blocked human monoclonal antibodies to the Rh(D) antigen of human red blood cells. In particular, it relates to such antibodies of the IgG3 subclass which may be used in a rapid and direct detection method of the Rh(D) antigen.

The Rhesus blood group system is a major antigenic constituent of the human red blood cell membrane; of this group, the Rh(D) antigen is of particular clinical importance in relation to isoimmune reactions. An Rh (D-) individual with anti-Rh(D) who receives Rh(D+) blood is liable to suffer substantial red blood cell (RBc) destruction due to the Rh(D) phenotype incompatibility, and thus blood of donors must routinely be classified as Rh(D+) or Rh(D-).

The Rh(D) antigen is also responsible for haemolytic disease of the newborn (HDN). This condition arises in newborn Rh(D+) infants of Rh(D-) mothers previously sensitised to Rh(D) antigen as a result of IgG anti-Rh(D) antibodies crossing the placenta during pregnancy and causing foetal RBC destruction. Sensitization of the Rh(D-) mother to Rh(D) antigen often occurs during the birth of an earlier Rh(D+) child due to some foetal RBCs entering the maternal circulation and being recognised as foreign by the maternal immune system. To reduce the incidence of HDN, it is routine practice in the United Kingdom and many other countries to give anti-Rh(D) antibodies to Rh(D-) mothers immediately after the birth of an Rh(D+) infant so that any Rh(D+) RBCs which may have entered the maternal circulation are rapidly removed (Mollison, P.L. (1983) Blood Transfusion is clinical Medicine, 7th Edn., Blackwell Scientific, Oxford; Laros Jr., R.K. (1986), "Erythroblastosis Fetalis" in "Blood Group Disorders in Pregnancy", Ch.7, p.103).

Since Rh(D) blood typing is so important, there is much interest in establishing a technique which is both rapid and reliable.

At the present time, two diagnostic protocols are in routine use. One test involves indirect agglutination with an anti-D IgG. These antibodies are known as "incomplete"; they fail to agglutinate Rh D(+ve) RBCs in saline suspension. Traditionally, this problem has been overcome by the addition of so-called potentiators. These are biological polymers, such as bovine albumin, or non-biological polymers such as polyvinylpyrrolidone or Ficoll, which modify the surface and permit the cells to be agglutinated by the IgG reagent. However, these potentiators sometimes give a false positive reaction by causing the non-specific agglutination of red blood cells coated with IgG, as occurs in certain diseases, for example autoimmune haemolytic anaemia. In consequence, a reagent control containing potentiator alone but no anti-D must be run in parallel with every test when using this type of reagent, which is costly both for manufacturer and user, since the tests require lengthy incubation.

In an alternative indirect agglutination method, a second reagent which can be used to agglutinate RBCs coated with anti-D is anti-immunoglobulin serum (Coomb's Reagent), which serves to cross link IgG molecules bound to different RBCs.

The other test utilises IgM antibodies against Rh(D), which due to their multivalent nature, are direct agglutinins. Naturally occurring polyclonal IgM anti-D's suffer from the disadvantages that the agglutination reaction is lengthy and they are difficult to obtain. Some human monoclonal IgM anti-D's are available and can be used as reagents but they suffer from the disadvantage that they react poorly or not at all with certain D variant red blood cells.

Therefore, it is of considerable medical importance that rapid and reliable blood typing procedures be found which avoid the necessity for the introduction of secondary reagents. It has been found that it is possible to speed both the reaction times and the accuracy of blood typing by converting incomplete polyclonal anti-D IgG to become direct agglutinins. This may be accomplished by the process of reduction of the disulphide bonds at the hinge region of the IgG molecule, as described by Romans et al. (Proc. Natl. Acad. Sci. 74 2531-5, 1977) who also noted that reduced antibody permitted to re-oxidise in air no longer behaved as a direct agglutinin and that this reversion to incomplete antibody could be prevented by S-alkylation. Their results suggested that mild reduction and alkylation of IgG resulted in increased flexibility of the molecule, such that intercellular bridging could occur, as further described by Michaelsen (Molec. Immunol. 25 639-46, (1988)).

US-4296090 discloses a reduced and S-alkylated polyclonal anti-Rh(D) IgG of high titer and its use as a reagent for rapid blood typing without the need for a potentiator. However, none of these cited publications specifically describes conversion of monoclonal antibodies to complete antibodies.

A monoclonal antibody is preferred to a polyclonal IgG in permitting a continual supply of blood-typing reagent having consistent properties, thus ensuring greater reliability and reproducibility of typing. At the present, reduced and S-alkylated Rh(D) blood typing reagents have been restricted to polyclonal antibodies, including all of IgG subclasses 1,2,3 and 4.

We have now investigated a series of 15 monoclonal anti-D antibodies of subclasses IgG1 and IgG3 to investigate the importance of subclass on the performance of reduced and alkylated anti-D reagents and have found that reduced and alkylated monoclonal IgG3 antibodies are much more effective in direct agglutination than reduced and alkylated monoclonal IgG1 antibodies of comparable potency prior to treatment. Although S-alkylation is a convenient method of preventing re-oxidation to normal IgG, it will be appreciated that other methods of S-blocking are equally effective.

Thus, according to one aspect, the present invention provides reduced and S-blocked anti-Rh(D) antibodies of the IgG3 subclass, preferably monoclonal antibodies, said reagent being useful for rapidly determining the RhD status of blood samples.

Reduction of the IgG3 antibody may be effected using mild reducing agents serving to reduce S-S bonds to SH, for example mercaptans such as dithiothreitol. Blocking of the SH groups produced by reduction may be effected using appropriate reagents which will normally be water-soluble. Blocking groups include alkyl groups (e.g. $C_{1-4}$ alkyl groups), acyl groups (e.g. $C_{1-4}$ alkanoyl groups) and other groups commonly used for S-blocking or S-protecting polypeptides, for example as described by Schroeder, E. and Lubke, K., The Peptides, Vols 1 and 2, Academic Press, New York and London, 1965 and 1966. A preferred S-blocking group is the group $-CH_2CONH_2$ which may be introduced by reaction of the reduced IgG3 with iodoacetamide. The blocking groups may be optionally substituted and in general alkyl groups will carry solubilising groupings such as carboxyl or carboxamido groups.

Thus, according to a further aspect, the present invention provides a process for the preparation of the antibodies of the present invention comprising reducing disulphide bonds of the antibody to sulphydryl groups and S-blocking the sulphydryl groups so formed.

We have found that reduced and S-blocked IgG3 Rh(D) antibodies are superior to reduced and S-blocked IgG1 antibodies in two major respects. Firstly, they produce much higher scores in direct agglutination tests, substantially equal to or superior to those in the indirect albumin test, while the corresponding IgGls give consistently lower scores, often zero. Secondly, the reduced and S-blocked IgGls are found to give a so-called prozoning effect at high antibody concentrations which renders them unreliable in Rh(D) blood typing; they are found to give an initial score in the direct agglutination test which reduces over a period of a few minutes to a lower value.

15 monoclonal anti-D antibodies were studied, 6 of the IgG3 subclass, referred to as A4, A5, A7, A8, E1, E2 and 9 of the IgG1 subclass A1, B7, B8, B11, B13, E3, E4, E5, E6. Many of these cell lines have been described in EP-A-87303629.7, WO 89/02442, WO 89/02600, WO 89/02443. The cell lines producing monoclonal antibodies referred to as A7 and B7 have been deposited at the European Collection of Animal Cell Cultures, Porton Down, UK, under accession numbers 87091606 and 87091603 on 16th September 1987.

While monoclonal antibodies are of particular interest our experiments have shown that polyclonal IgG3 anti-D antibodies when reduced and alkylated show the same advantages over reduced and alkylated IgG1 antibodies as are demonstrated by the corresponding monoclonals. Polyclonal IgG3 anti-D antibodies may be obtained by selective affinity chromatography of polyclonal IgG containing subclasses 1,2,3 and 4.

Tissue culture supernatants from these antibody producing cell lines contain varying concentrations of monoclonal anti-D antibody, of varying affinity. Each was titrated in its untreated state by an albumin displacement technique in which antibodies agglutinate positive cells in a milieu of high albumin concentration. No subclass correlation was observed (Table 1). The antibodies were reduced with dithiothreitol, alkylated with iodoacetamide and then titrated as direct agglutinins of RBCs in saline suspension (Table 1).

In order to eliminate bias caused by variations in antibody concentration and affinity, the antibodies were ranked according to the ratio calculated between titration score after reduction and alkylation to that before such treatment but with the addition of albumin. Table 1 reveals a very clear difference between IgG3 and IgG1. IgG3s producing consistently high scores substantially equal to or greater than those using the indirect albumin method, while IgG1s produced much lower scores than in the indirect method (often zero). Details of the scoring system are given in Example 1 hereinafter.

In order to take account of any changes in antibody concentration or affinity that may be caused by the reduction and alkylation procedures, the antibodies were also tested following treatment for their ability to agglutinate bromelain treated RBCs in saline. This proteolytic enzyme renders RBCs agglutinable by incomplete antibodies and therefore any agglutination reaction observed is independent of the reduction and alkylation process. Such ranking was independent of subclass (Table 1) although it was in a different order to ranking by albumin displacement of untreated cells. Likewise the ratio of titration score before reduction and alkylation (i.e. by albumin displacement) to that after reduction and alkylation (of bromelain treated cells) showed no correlation with antibody subclass.

## Table 1

### Reactions of Monoclonal Antibodies before and after reduction and alkylation

| Antibody | Subclass | Reduced and alkylated. Direct agglutination | Titration Score Untreated. Albumin displacement | Reduced and alkylated. Bromelain treated | Ratio R+A/albumin | Subclass Average R+A/albumin | Ratio bromelain/ albumin |
|---|---|---|---|---|---|---|---|
| A4 | IgG3 | 41 | 32 | 57 | 1.3 | | 1.8 |
| A7 | IgG3 | 46 | 42.5 | 69 | 1.1 | | 1.6 |
| A8 | IgG3 | 60 | 57 | 67 | 1.1 | 1.03 | 1.2 |
| E1 | IgG3 | 28 | 31.5 | 58 | 0.9 | | 1.8 |
| E2 | IgG3 | 70 | 75 | 84 | 0.9 | | 1.1 |
| A5 | IgG3 | 40.5 | 44 | 71 | 0.9 | | 1.6 |

EP 0 489 771 B1

| | B13 | E3 | B11 | E4 | A1 | E5 | B7 | E6 | B8 |
|---|---|---|---|---|---|---|---|---|---|
| | 1.6 | 1.9 | 1.4 | | 1.8 | | 1.8 | 1.8 | 1.5 |
| | | | | 0.25 | | | | | |
| | 0.5 | 0.5 | 0.5 | 0.4 | 0.3 | 0.1 | 0 | 0 | 0 |
| | 88 | 48 | 80 | | 75 | | 45 | 42 | 32 |
| | 56 | 25 | 57 | 78 | 42 | 61 | 24.5 | 23 | 21 |
| | 29 | 12 | 27 | 33 | 12 | 4 | 0 | 0 | 0 |
| | IgG1 | IgG1 | IgG1 | IgG1 | IgG1 | IgG1 | IgG1 | IgG1 | IgG1 |

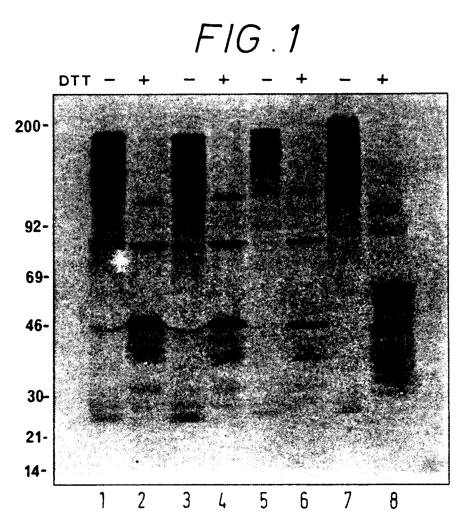

Figure 1, which shows an analysis of some of the monoclonal antibodies both before and after reduction and alkylation, by non-reducing polyacrylamide gel electrophoresis and immunoblotting confirms that the reduction and alkylation processes are effective. Before reduction and alkylation, all antibodies showed heavy chain staining at around 150,000-170,000 molecular weight, which is consistent with intact IgG. All four antibodies showed complete separation of heavy chains after reduction and alkylation, with the heavy chain of IgG1 now migrating at around 50,000 molecular weight, and that of the IgG3 at around 65,000.

As indicated above, we have found a further difference between the IgG subclasses which is relevant to the usefulness of anti-Rh(D) IgG, when reduced and alkylated, at agglutinating RBCs. This is the fact that reduced and alkylated IgG1s are subject to a prozone effect. This effect occurs at high antibody concentrations and results in a diminution of expected agglutination strength with increased time of incubation in direct agglutination tests. In an experimental red cell antibody dilution series, high agglutination grades of red cells are expected at high concentrations of antibody, which diminish as the dilution of the antibody increases. However, if antibody is present in excess of the available antigen on red cells, a lower agglutination grade can sometimes be observed at high antibody concentration. In this situation, there is enough antibody to saturate the available antigen on red cells by binding monovalently, such that cross-linking and therefore agglutination cannot occur. Table 2 shows the development of prozones with 15 minutes incubation in direct agglutination with the 15 monoclonal antibodies. Only one of the IgG3 antibodies (A8) shows a very slight prozone; by contrast, seven of the nine IgG1s show marked prozones. The other two IgG1s showed no, or weak reaction. Details of the scoring system used are given in Example 1 hereinafter.

## Table 2
## Development of prozones with reduced and alkylated monoclonal IgG

| Antibody | Subclass | Incubation Time | Dilution Titration | | | | | | | | | | Total |
|---|---|---|---|---|---|---|---|---|---|---|---|---|---|
| | | mins | 2 | 4 | 8 | 16 | 32 | 64 | 128 | 256 | 512 | 1024 | |
| E1 | IgG3 | 1 | 6 | 5 | 3 | 1 | 0 | 0 | 0 | 0 | 0 | 0 | 15 |
| | | 15 | 6 | 6 | 4.5 | 3 | 3.5 | 1 | 0 | 0 | 0 | 0 | 24 |
| A7 | IgG3 | 1 | 6 | 6 | 5 | 4 | 2 | 1 | 0 | 0 | 0 | 0 | 24 |
| | | 15 | 6 | 6 | 6 | 5 | 2 | 1.5 | 0 | 0 | 0 | 0 | 26.5 |
| A5 | IgG3 | 1 | 6 | 5 | 4.5 | 3 | 2 | 1 | 0.5 | 0 | 0 | 0 | 22 |
| | | 15 | 6 | 6 | 6 | 4.5 | 3.5 | 1.5 | 0 | 0 | 0 | 0 | 27.5 |
| A4 | IgG3 | 1 | 6 | 6 | 5 | 3 | 1.5 | 0 | 0 | 0 | 0 | 0 | 21.5 |
| | | 15 | 6 | 6 | 5.5 | 4 | 2.5 | 0.5 | 0 | 0 | 0 | 0 | 24.5 |

| Clone | Isotype | Dose | | | | | | | | | | | Total |
|---|---|---|---|---|---|---|---|---|---|---|---|---|---|
| E2 | IgG3 | 1 | 6 | 6 | 6 | 5.5 | 5 | 4.5 | 2.5 | 0 | 0 | 0 | 35.5 |
| | | 15 | 5.5 | 5.5 | 5 | 5 | 5.5 | 5.5 | 3.5 | 2 | 1 | 0 | 38.5 |
| A8 | IgG3 | 1 | 6 | 6 | 6 | 5 | 3.5 | 2.5 | 1.5 | 1 | 0 | 0 | 31.5 |
| | | 15 | 5 | 6 | 6 | 6 | 6 | 5 | 3 | 2 | 1 | 0 | 40 |
| B11 | IgG1 | 1 | 5.5 | 4 | 2.5 | 2 | 1 | 0 | 0 | 0 | 0 | 0 | 15 |
| | | 15 | 1 | 2.5 | 2.5 | 1 | 1 | 0 | 0 | 0 | 0 | 0 | 8 |
| A1 | IgG1 | 1 | 4.5 | 2 | 0 | 0 | 0 | 0 | 0 | 0 | 0 | 0 | 6.5 |
| | | 15 | 3.5 | 3.5 | 2 | 1.5 | 1 | 0 | 0 | 0 | 0 | 0 | 11.5 |
| B13 | IgG1 | 1 | 4.5 | 4 | 3 | 2 | 1.5 | 1 | 1 | 0 | 0 | 0 | 17 |
| | | 15 | 3 | 1.5 | 2.5 | 2 | 1.5 | 1.5 | 1 | 1 | 0 | 0 | 12 |
| E4 | IgG1 | 1 | 5 | 4.5 | 3.5 | 2.5 | 1.5 | 1 | 0 | 0 | 0 | 0 | 18 |
| | | 15 | 2.5 | 3 | 4.5 | 5 | 5.5 | 3.5 | 2.5 | 2 | 1 | 0 | 29.5 |

EP 0 489 771 B1

| | B7 | | B8 | | E6 | | E5 | | E3 |
|---|---|---|---|---|---|---|---|---|---|
| | IgG1 | | IgG1 | | IgG1 | | IgG1 | | IgG1 |
| | 1 | 15 | 1 | 15 | 1 | 15 | 1 | 15 | 1 | 15 |
| 0 | 2.5 | 0 | 2 | 0 | 0 | 3.5 | 8 | 7 | 7.5 |
| 0 | 2.5 | 0 | 0 | 0 | 0 | 0 | 0 | 0 | 0 |
| 0 | 0 | 0 | 0 | 0 | 0 | 0 | 0 | 0 | 0 |
| 0 | 0 | 0 | 0 | 0 | 0 | 0 | 0 | 0 | 0 |
| 0 | 0 | 0 | 0 | 0 | 0 | 0 | 0 | 0 | 0 |
| 0 | 0 | 0 | 0 | 0 | 0 | 0 | 0 | 0 | 0 |
| 0 | 0 | 0 | 0 | 0 | 0 | 0 | 1.5 | 0 | 1 |
| 0 | 1 | 0 | 0 | 0 | 0 | 0 | 2.5 | 0 | 1 |
| 0 | 1.5 | 0 | 0 | 0 | 0 | 1 | 2.5 | 1.5 | 2 |
| 0 | 0 | 0 | 1 | 0 | 0 | 1 | 1.5 | 2 | 2.5 |
| 0 | 0 | 0 | 1 | 0 | 0 | 1.5 | 0 | 3.5 | 1 |

It will be appreciated that in routine blood typing, only a single dilution is appropriate on economic grounds, so that a false low value would not be detected due to prozoning.

In routine blood testing, it has become apparent that there are several variants on the D antigen. Blood typing by agglutination with anti Rh(D) divides blood types into either Rh(D+) or Rh(D-) on the apparent presence/absence of Rh(D) antigen. However, a small number of persons with apparently Rh(D-) blood have RBCs that are not directly agglutinated by anti-Rh(D) during such routine testing, but that do react when the D-typing test is performed using selected anti-Rh(D) reagents by the indirect antiglobulin test. Cells thus identified are designated D$^u$. The frequency of the D$^u$ phenotype is about 0.2% overall, 0.6% among Caucasians, and about 1.5% of all Rh(D-) gravid women. At least three different mechanisms may be responsible for the expression of the D$^u$ phenotype: (1) hereditary absence of a portion of the complete Rh(D) antigen, (2) gene interaction with suppression of Rhesus gene D by C in the trans position, and (3) a D gene producing a weak antigen.

In the early 1950s, reports first appeared of the presence of anti-Rh(D) in individuals of the D$^u$ phenotype following blood transfusion with Rh(D+) blood or pregnancy resulting in the birth of a Rh(D+) infant. It later became apparent that in some individuals whose blood is classified Rh(D+) parts of the Rh(D) antigen are missing from the RBCs. When exposed by transfusion or pregnancy to Rh(D+) RBCs carrying the complete Rh(D) antigen, persons carrying an incomplete Rh(D) antigen on their RBCs are capable of making alloanti-D against the Rh(D) antigen portion they lack. The blood of such individuals is called D variant when the RBCs react directly with routine anti-Rh (D) reagents or D$^u$ variant when the cells react only by the indirect antiglobulin technique.

The observation that allo anti-Rh(D) can be produced in patients who have Rh(D+) RBCs has led to common usage of the term "D mosaic" to describe the Rh(D) antigen in its complete native form. Routine anti-Rh(D) reagents generally cannot differentiate those RBCs that lack part of the D mosaic from those that have all the D components.

The D variant phenotypes have been categorised by Tippett and Sanger (Vox. Sang. (1962)7, 9-13). This system is based on the interaction of RBCs and serum from D(-ve) and D$^u$ variant individuals. The six categories (see Table 3 below) allow for expansion; subdivisions are already recognised in categories III, IV and V. Categories I and II have been found to have so many similarities that they are now generally considered as a single sub-group.

## Table 3

### Tippett and Sanger categories for D- or D$^u$ positive blood with anti-Rh(D)

| Category | Racial Origin | Usual haplotype[1] |
|---|---|---|
| I | White | DCe |
| II | | |
| IIIa | Black | |
| IIIb | Usually Black | Dce |
| IIIc | White | |
| IVa | Mostly Black, some White | |
| IVb | White | Dce |
| Va | Black and White | |
| Vb | White | D$^u$Ce |
| Vc | Black and White | |
| VI | Nearly all White | D$^u$Ce |

[1]Haplotype refers to the three pairs of allelic genes, C-c, D-d and E-e, which act at very closely linked loci, which define the Rh phenotype of RBCs according to the Fisher-Race system.

An alternative, but lesser used, classification by Wiener uses letters A,B,C,D instead of Roman Numerals. Although there is no direct correlation between the two systems, it is often considered that $D^B$ and $D^{VI}$ are interchangeable.

Although the frequency of D and $D^u$ variant individuals within the human population is relatively low, the total number of individuals of these blood types who potentially have some risk of effective anti-Rh(D) formation as a result of exposure by blood transfusion or pregnancy to non-variant Rh(D+) cells is far from insignificant.

Since it is found that there are a number of variants of D(+ve) cells which do not always give a positive result, it is desirable that any blood typing antibody reagent should be able to deal with such variants. In general, therefore, a reduced and alkylated IgG3 according to the invention will desirably be used in admixture with one or more further anti-D monoclonal antibodies having one or more additional binding specificities as described below.

There are two types of variant D(+ve) cells. Low dose cell types, termed $D^u$, are quantitative variants. They cannot all be detected by direct agglutination with presently available reagents, which are either IgMs, which cannot be taken through to an antiglobulin test to detect these variants as there is no anti-IgM component in the conventional antiglobulin reagents used, or IgGs, which can only be utilised in conjunction with albumin or other potentiators. Thus the reduced and S-blocked IgG3 molecules according to the invention have the advantage of being usable in the direct agglutination test to detect RhD(+ve)s and can then be used to distinguish $D^u$ variants from true D(-ve)s in an in situ antiglobulin test.

The second type of variant RhD(+ve) is the qualitative variant. The D antigen is composed of various epitopes. Some individuals lack some of these epitopes and so have a partial D antigen, but at normal dose. However, because they lack a portion of the D antigen, they can make antibody to this portion, and, if mistyped as D negative, qualitative D variant cells can elicit a strong antibody response in D(-ve) individuals.

We have raised IgG3 monoclonal antibodies that recognise all known D-variant RBCs, except for category $D^{VI}$, for which we have an IgG1 monoclonal. It should be emphasised that $D^{VI}$ variant cells are rare.

Therefore, according to a further aspect, the invention provides a blend of monoclonal antibodies capable of recognising substantially all Rh(D) variants, said blend consists of a reduced and alkylated IgG3 that in an antiglobulin test detects all variants apart from $D^{VI}$, with an unreduced IgG1 that detects $D^{VI}$ variants. Amongst such antibodies, those particularly preferred include A7, A8 and B7.

According to another aspect, the invention provides a method of use of this antibody blend, said method comprising a direct agglutination test, whereby all normal D(+ve) cells, "high grade" (borderline) $D^u$'s and variants other than $D^{VI}$ are detected by immediate spin. Any negatives are subsequently taken through an antiglobulin test where the IgG3 and IgG1 components detect $D^u$ variants and the IgG1 component detects $D^{VI}$ variants.

When red cells have been incubated with antibody, the cells must normally be washed before adding antiglobulin reagent. This is because otherwise free immunoglobulin from the antibody containing reagent will neutralise the antiglobulin reagent and no agglutination of antibody coated cells will occur. When using polyclonal anti-D for the detection of $D^u$'s in an antiglobulin test, it is necessary to wash the red cells three times after incubation with the anti-D before addition of antiglobulin. This is because the polyclonal reagent contains high levels of normal serum immunoglobulin which does not react with the red cells. We have found that the blended monoclonal reagent of the invention has considerable advantages in this respect since all the immunoglobulin in the reagent is of anti-D specificity and therefore most of it will react with D(+ve) cells. Little free immunoglobulin will remain after incubation.

We have compared our reduced and alkylated blended monoclonal anti-D reagent with a commercial anti-D reagent which contains polyclonal anti-D, using zero, one and three washes prior to antiglobulin addition. The titration scores, shown in Table 4 below, show that whereas the polyclonal containing reagent needs three washes prior to antiglobulin addition, our monoclonal reagent requires only one wash. This gives our reagent significant efficiency advantages in $D^u$ testing.

## Table 4

### Number of washes required before proceeding to antiglobulin addition when using reduced and alkylated blended monoclonal anti-D

| | | | Dilution of reagent | | | | | | | | | |
|---|---|---|---|---|---|---|---|---|---|---|---|---|
| washes | reagent* | cell | N | 2 | 4 | 8 | 16 | 32 | 64 | 128 | 256 | total |
| 0 | P | $D^u$ | 0 | 0 | 0 | 0 | 0 | 0 | 0 | 0 | 0 | 0 |
| 0 | M | $D^u$ | 0 | 0 | 3 | 4 | 3 | 2 | 2 | 1 | 0 | 15 |
| 0 | P | $D^{vi}$ | 0 | 0 | 0 | 0 | 0 | 0 | 0 | 0 | 0 | 0 |
| 0 | M | $D^{vi}$ | 6 | 5 | 5 | 5 | 4 | 3 | 3 | 1 | 0 | 32 |
| 1 | P | $D^u$ | 2 | 4 | 5 | 3 | 3 | 2 | 1 | 0 | 0 | 20 |
| 1 | M | $D^u$ | 6 | 6 | 6 | 5 | 4 | 3 | 3 | 2 | 1 | 36 |
| 1 | P | $D^{vi}$ | 3 | 4 | 3 | 1 | 0 | 0 | 0 | 0 | 0 | 11 |
| 1 | M | $D^{vi}$ | 6 | 5 | 5 | 5 | 4 | 3 | 3 | 1 | 0 | 32 |
| 3 | P | $D^u$ | 6 | 5 | 5 | 4 | 3 | 1 | 0 | 0 | 0 | 24 |
| 3 | M | $D^u$ | 6 | 6 | 5 | 4 | 4 | 3 | 2 | 2 | 1 | 33 |
| 3 | P | $D^{vi}$ | 5 | 5 | 4 | 3 | 2 | 1 | 0 | 0 | 0 | 20 |
| 3 | M | $D^{vi}$ | 6 | 6 | 6 | 5 | 4 | 3 | 2 | 0 | 0 | 32 |

* Reagent – P = polyclonal reagent; M= R&A blended monoclonal reagent

The efficacy of such an antibody blend is demonstrated in Table 5, using a series of cells of known RhD subclass. Cells expressing normal Rh-D antigen gain high scores with the blend in direct agglutination, whilst $D^u$ and $D^{VI}$ variants are not detected. However, these variants are detected upon indirect agglutination with the antibody blend together with antiglobulin (AGT). It should be noted that these variants are not detected at either stage by the positive control IgM anti-D, since this reagent does not recognise such D variants and the antiglobulin reagent used in the indirect agglutination is specific to IgG.

## Table 5

### Assessment of blend of reduced and alkylated IgG3 anti D with IgG1 anti D

| | Titration Score | | | | | |
| | Spun at 20°C | | | AGT | | |
| Cell | T | S | A B | T | S | A B |
|---|---|---|---|---|---|---|
| R1r | 5 | 6 | 0 | | | 0 |
| R1r | 5 | 5 | 0 | | | 0 |
| R1r | 5 | 6 | 0 | | | 0 |
| R1r | 5 | 5 | 0 | | | 0 |
| R2r | 5 | 6 | 0 | | | 0 |
| R2r | 5 | 6 | 0 | | | 0 |
| $D^u$ | 0 | 0 | 0 | 5 | 1 | 0 |
| $D^u$ | 2 | 1 | 0 | 5 | 1 | 0 |
| $D^u$ | 0 | 0 | 0 | 4 | 0 | 0 |
| $D^{VI}$ | 0 | 0 | 0 | 2 | 0 | 0 |
| $D^{VI}$ | 0 | 0 | 0 | 3 | 0 | 0 |
| $D^{VI}$ | 0 | 0 | 0 | 2 | 0 | 0 |
| rr | 0 | 0 | 0 | 0 | 0 | 0 |

T  = Test sample

S  = Standard positive control (IgM anti-D)

AB = inert AB serum (negative control)

According to a still further aspect of the present invention we provide a pharmaceutical composition comprising at least one reduced and S-blocked antibody of the present invention together with at least one pharmaceutical carrier or excipient for use in passive immunisation of an Rh(D-) or $D^u$ variant mother after the birth of an Rh(D+) child to prevent sensitisation of the mother to the Rh(D) antigen. To provide a highly efficient prophylactic preparation for use in the prevention of HDN, a monoclonal antibody of the present invention may be employed with one or more further anti-Rh(D) antibodies.

Further details of the reagents are provided in the following non-limiting Examples.

Example 1

(i) Monoclonal Antibodies

(a) Monoclonal Antibody producins cell lines

All antibodies used were from human monoclonal cell lines grown in tissue culture according to standard procedures as described in EP-A-87303629.7, GB-A-8722018, GB-A-8722019 and GB-8722020.

Monoclonal antibodies of subclass IgG3 were A4, A5, A7, A8, E1, E2 and those of subclass IgG1 were A1, B7, B8, B11, E3, E4, E5, E6. Antibodies A1, A4, A5, A7, A8, B7, B8, B11, B13, E1, E3, E6 were from EBV (Epstein Barr Virus) transformed human peripheral blood lymphocytes, produced by Kumpel et al (Kumpel, B.M., Poole, G.D. and Bradley, B.A., Brit.J. Haematol 71, 125-129 (1989)). Antibodies E2, E4, E5 were from human EBV transformed lymphocytes fused with a mouse myeloma cell line and were produced by Thompson et al (Thompson, K.M., Hough, D.W., Maddison, P.J., Melamed, M.D. and Huges-Jones, N. J. Immunol. Meth. 94 7-12 (1986)).

(b) Reduction and alkylation of IgG.

An equal volume of saturated ammonium sulphate solution was added to anti-D tissue culture supernatant whilst stirring and left for one hour at room temperature with gentle stirring. (Alternatively solid ammonium sulphate was added to the supernatant whilst vigorously stirring to give a final saturation of ammonium sulphate of 50%). The preparation was centrifuged at 3900 g for 10 minutes. The precipitate was washed twice in 50% saturated ammonium sulphate, and then redissolved in 1/5 of starting supernatant volume of 0.5 M Tris/HCl buffer pH 8.0.

110 μl/ml IgG solution of freshly prepared 0.1 M dithiothreitol (DTT) in 0.5 M Tris/HCl buffer was added, mixed and incubated for one hour at room temperature. 110 μl/ml IgG solution of freshly prepared 0.21 M iodoacetamide (IAA) in the Tris buffer was added, mixed and incubated for 30 min at 4°C. The resultant solution was dialysed against phosphate buffered saline pH 7.2 (PBS). 30% bovine serum albumin (BSA) was added to give a final BSA concentration of 2.5%.

(ii) Serology

(a) Direct Agglutination Method

Fifty microlitres of thrice washed 3% red cells in PBS were added to 100 μl of the sample to be tested in a 12x75 mm glass tube and the tube incubated for one minute at 37°C and then immediately centrifuged at 110 g for one minute. The cell button was gently dislodged from the base of the tube and the cells resuspended by gentle shaking. The degree of agglutination was graded on a scale from 0 to 6, grades 1 and 2 being visible microscopically only.

(b) Albumin Displacement Method

Fifty microlitres of thrice washed 3% red cells in PBS were added to 100 μl of the sample to be tested in a 12 x 75 mm glass tube and the tube incubated for one minute at 37°C and then immediately centrifuged at 110 g for one minute. The tubes were tilted and 50 μl of 30% BSA run down the side of the tubes to layer over the cell buttons. Tubes were incubated for a further 15 mins at 37°C, without mixing, and then the degree of agglutination assessed as above.

(c) Bromelain Treatment

0.1 g of bromelain (Hughes & Hughes) was added to 20 ml of PBS. The suspension was stirred for 5 mins at room temperature and then centrifuged at 2100 g for 2 mins. The supernatant was decanted off and made up to 100 ml with PBS. Test red cells were suspended at 3% in the bromelain solution for 15 mins at room temperature. The cells were washed twice with PBS and resuspended to 3% in PBS. The bromelain treated red cells were then used as per the direct agglutination method above.

Samples were tested in these three techniques by titration (from 1/2 to 1/1024, dilutions in PBS containing 2.5% BSA) against group $OR_1r$ cells and undiluted against group Orr cells. The titration score was obtained for each antibody in each technique by adding the agglutination grades for each dilution together.

(d) Electrophoresis and Immunoblotting

Samples of IgG before and after reduction and alkylation were analyzed under non-reducing conditions on 3-15% linear gradient polyacrylamide gels, using the discontinuous buffer system (Laemmli, U.K. (1970) Nature 227 680-684). After electrophoresis, the separated proteins were electroblotted into nitrocellulose (Towbin et al., (1979) Proc. Natl. Acad. Sci. USA 76 4350-4354), and immunoperoxidase stained for the heavy chain of IgG using peroxidase conjugated anti-human IgG Fc (Serotec) and diaminobenzidine substrate. The resulting blot is shown in the attached Figure 1 in which lanes 1 and 2: antibody E4 (IgG1), lanes 3 and 4: antibody E5 (IgG1), lanes 5 and 6: antibody B7 (IgG1) and lanes 7 and 8: antibody A8 (IgG3). Lanes 1,3,5 and 7 are prior to reduction and alkylation and lanes 2,4,6 and 8 are after reduction and alkylation and molecular weights (x 1000) are indicated adjacent to lane 1.

Example 2

Solution for Rh(D) typing of RBCs

In general, it is preferred to use a blend of an anti-Rh(D) monoclonal IgG3 antibody according to the invention (e.g. A8) with a further IgG1 monoclonal antibody having anti D$^{VI}$ activity, for example B7.

Solution for Manual Use

Final blends which may be used are
1. 9:1 reduced and blocked A8:B7
2. 9:1 reduced and blocked A7:B7
These blends can be used in all manual tests for D and D$^{u}$ typing, e.g. tube or microplate.

Example 3

Subclass Dependence of the Reduction and Alkylation of Polyclonal IgG anti-D

The results obtained with the panel of monoclonal antibodies predict that the important component of reduced and alkylated polyclonal IgG anti-D sera is the IgG3. Previous studies by Romans et al (Proc. Natl. Acad. Sci. 74 2531-5 (1977)) and US-4296090 have not addressed the subclass of the antibodies.

We have separated human polyclonal IgG anti-D into IgG1,2 and 4 and IgG3 rich fractions by affinity chromatography on Protein G sepharose (which binds all IgG subclasses) and Protein A sepharose (which binds all IgG subclasses except IgG3). The purity of the fractions was assessed by polyacrylamide gel electrophoresis under reducing conditions, and stained for total protein with coomassie blue. The heavy chains of IgG1,2 and 4 are all of the same molecular weight (approx 50000) whereas the heavy chain of IgG3 is significantly heavier (approximately 60000). Light chains of all subclasses are the same molecular weight (27000). Densitometric scans of the gels showed that the IgG1,2,4 fraction did not contain any demonstrable IgG3, but the IgG3 fraction contained approximately 30% contamination with IgG1,2 or 4.

Fractions were assessed before and after reduction and alkylation by the same methods as for the monoclonal antibodies. The titration scores for various antibody dilutions, shown in Table 6, show that, as for the monoclonal antibodies, the IgG3 rich fraction gives a higher ratio of serological activity post treatment to pretreatment than the IgG1,2,4 fraction.

Our observations on the subclass dependence of the performance of reduced and alkylated monoclonal IgG anti-D reagents therefore also apply to reduced and alkylated polyclonal IgG anti-D reagents.

15

## TABLE 6
### Reactions of polyclonal antibodies before and after reduction and alkylation

Titration Score
Dilution

RATIO

| FRACTION | TECHNIQUE | TIME | N | 5 | 10 | 20 | 40 | 80 | 160 | 320 | 640 | 1280 | TOTAL | 1 | 2 | 3 |
|----------|-----------|------|---|---|----|----|----|----|-----|-----|-----|------|-------|---|---|---|
| IgG1,2,4 | ALBUMIN | 15MIN | 6 | 6 | 5 | 5 | 4.5 | 3.5 | 3 | 3 | 2 | 1 | 39 | 0.3 | | |
| IgG3 | ALBUMIN | 15MIN | 6 | 6 | 5 | 4 | 3 | 1.5 | 1 | 0 | 0 | 0 | 26.5 | 0.6 | | |
| IgG1,2,4 | ENZYME | 1MIN | 6 | 6 | 6 | 5 | 4.5 | 4.5 | 4.5 | 4.5 | 4.5 | 3.5 | 49 | | 0.2 | |
| IgG3 | ENZYME | 1MIN | 3.5 | 4 | 3.5 | 3.5 | 3 | 3 | 2.5 | 2.5 | 2.5 | 2 | 30 | | 0.4 | |
| IgG1,2,4 | ENZYME | 15MIN | 6 | 6 | 6 | 6 | 6 | 6 | 6 | 6 | 5 | 5 | 58 | | 0.2 | |
| IgG3 | ENZYME | 15MIN | 3.5 | 4 | 3 | 3 | 3 | 3 | 3 | 2.5 | 2.5 | 2 | 29.5 | | 0.6 | |
| R&A IgG1,2,4 | DIRECT | 1MIN | 6 | 3.5 | 1.5 | 0 | 0 | 0 | 0 | 0 | 0 | 0 | 11 | | 0.2 | 0.3 |
| R&A IgG3 | DIRECT | 1MIN | 6 | 4 | 2.5 | 0.5 | 0 | 0 | 0 | 0 | 0 | 0 | 13 | | 0.4 | 0.6 |
| R&A IgG1,2,4 | DIRECT | 15MIN | 3 | 3 | 3 | 3 | 1 | 0 | 0 | 0 | 0 | 0 | 13 | 0.3 | 0.2 | 0.2 |
| R&A IgG3 | DIRECT | 15MIN | 5.5 | 5.5 | 3.5 | 1.5 | 0 | 0 | 0 | 0 | 0 | 0 | 16 | 0.6 | 0.6 | 0.6 |
| R&A IgG1,2,4 | DIRECT | 60MIN | 1.5 | 2 | 3 | 3 | 2 | 0 | 0 | 0 | 0 | 0 | 11.5 | | | 0.2 |
| R&A IgG3 | DIRECT | 60MIN | 4.5 | 5.5 | 3.5 | 2 | 1 | 0 | 0 | 0 | 0 | 0 | 16.5 | | | 0.7 |
| R&A IgG1,2,4 | ENZYME | 1MIN | 6 | 6 | 5 | 5 | 4.5 | 4 | 3.5 | 3.5 | 3 | 3 | 43.5 | | | 0.3 |
| R&A IgG3 | ENZYME | 1MIN | 5.5 | 4.5 | 3 | 3 | 2.5 | 2 | 1.5 | 1 | 0 | 0 | 23 | | | 0.6 |
| R&A IgG1,2,4 | ENZYME | 15MIN | 6 | 6 | 6 | 6 | 6 | 6 | 5 | 5 | 4.5 | 3.5 | 54 | | | 0.2 |
| R&A IgG3 | ENZYME | 15MIN | 6 | 5 | 3.5 | 3 | 3 | 2 | 1.5 | 1 | 0 | 0 | 25 | | | 0.6 |
| R&A IgG1,2,4 | ENZYME | 60MIN | 6 | 6 | 6 | 6 | 5.5 | 5.5 | 5 | 4.5 | 4 | 3.5 | 52 | | | 0.2 |
| R&A IgG3 | ENZYME | 60MIN | 5.5 | 3 | 3 | 3 | 3 | 2.5 | 2 | 1 | 0 | 0 | 23 | | | 0.7 |

Ratio 1 = R+A/Albumin
Ratio 2 = R+A/Enzyme
Ratio 3 = R+A/R+A Enzyme

**Claims**

1. A reduced and S-blocked human anti-Rh(D) antibody of the IgG3 subclass.

2. An antibody as claimed in claim 1 being a monoclonal antibody.

3. An antibody as claimed in claim 1 or claim 2 being a reduced and S-blocked form of the monoclonal antibody produced by the cell line ECACC 87091606.

4. An antibody as claimed in any one of claims 1,2 or 3 wherein the S-blocking groups are optionally substituted alkyl or acyl groups.

5. An antibody as claimed in claim 4 wherein the S-blocking group is -$CH_2CONH_2$.

6. An anti-Rh(D) reagent comprising a monoclonal antibody as claimed in claim 2 in combination with one or more further anti-Rh(D) antibodies having one or more additional binding specificities.

7. An anti-Rh(D) reagent as claimed in claim 6 comprising a monoclonal antibody which recognises all Rh(D) variants apart from $D^{VI}$ and an unreduced IgG1 monoclonal antibody with anti-$D^{VI}$ activity.

8. A reagent as claimed in claim 7 wherein the IgG1 monoclonal antibody is that produced by the cell line ECACC 87091603.

9. A method of Rh-typing wherein an antibody as claimed in claim 1 is used in a direct agglutination test.

10. A method of Rh-typing wherein the reagent as claimed in claim 7 is used in a direct agglutination test and negative samples are subsequently taken through an antiglobulin test whereby $D^u$ and $D^{VI}$ variants are detected.

11. A method of Rh-typing as claimed in claim 10 wherein red cells are given a single wash following the direct agglutination test prior to the antiglobulin test.

12. A pharmaceutical composition for use in passive immunisation to prevent haemolytic disease of the newborn comprising at least one antibody according to claim 1 or claim 2 in association with a physiologically acceptable carrier or diluent.

13. A process for the preparation of the antibody as claimed in claim 1 comprising reducing disulphide bonds thereof to sulphydryl groups and S-blocking the sulphydryl groups so formed.

14. A process as claimed in claim 13 wherein the reducing agent is water soluble.

15. A process as claimed in claim 13 wherein the S-blocking group is an optionally substituted alkyl or acyl group.


**Patentansprüche**

1. Reduzierter und am S blockierter menschlicher Anti-Rh(D)-Antikörper der Subklasse IgG3.

2. Antikörper nach Anspruch 1, der ein monoclonaler Antikörper ist.

3. Antikörper nach Anspruch 1 oder 2, der eine reduzierte und am S blockierte Form des von der Zellinie ECACC 87091606 produzierten monoclonalen Antikörpers ist.

4. Antikörper nach einem der Ansprüche 1, 2 oder 3, worin die am S blockierenden Gruppen gegebenenfalls substituierte Alkyl- oder Acylreste sind.

5. Antikörper nach Anspruch 4, worin die am S blockierende Gruppe -$CH_2CONH_2$ ist.

6. Anti-Rh(D)-Reagenz, umfassend einen monoclonalen Antikörper nach Anspruch 2 in Kombination mit einem oder mehreren weiteren Anti-Rh(D)-Antikörpern mit einer oder mehreren zusätzlichen Bindungsspezifitäten.

7. Anti-Rh(D)-Reagenz nach Anspruch 6, umfassend einen monoclonalen Antikörper, der alle Rh(D)-Varianten außer D$^{VI}$ erkennt, und einen nichtreduzierten monoclonalen IgG1-Antikörper mit Anti-D$^{VI}$-Aktivität.

8. Reagenz nach Anspruch 7, worin der monoclonale IgG1-Antikörper der von der Zellinie ECACC 87091603 produzierte ist.

9. Verfahren zur Rh-Typisierung, worin ein Antikörper nach Anspruch 1 in einem direkten Agglutinationstest verwendet wird.

10. Verfahren zur Rh-Typisierung, worin das Reagenz nach Anspruch 7 in einem direkten Agglutinationstest verwendet wird, und negative Proben anschließend einem Antiglobulintest unterworfen werden, mit dem die D$^u$- und D$^{VI}$-Varianten nachgewiesen werden.

11. Verfahren zur Rh-Typisierung nach Anspruch 10, worin die Erythrocyten nach dem direkten Agglutinationstest vor dem Antiglobulintest einmal gewaschen werden.

12. Pharmazeutisches Präparat zur Verwendung zur passiven Immunisierung zur Verhütung der Erythroleukoblastose bei Neugeborenen, umfassend mindestens einen Antikörper nach Anspruch 1 oder 2 zusammen mit einem physiologisch verträglichen Träger oder Verdünnungsmittel.

13. Verfahren zur Herstellung des Antikörpers nach Anspruch 1, wobei man die Disulfidbindungen davon zu Sulfhydrylgruppen reduziert und die so gebildeten Sulfhydrylgruppen am S blockiert.

14. Verfahren nach Anspruch 13, worin das Reduktionsmittel wasserlöslich ist.

15. Verfahren nach Anspruch 13, worin die am S blockierende Gruppe ein gegebenenfalls substituierter Alkyl- oder Acylrest ist.

**Revendications**

1. Anticorps anti-Rh(D) humain réduit et S-bloqué de la sous-classe IgG3.

2. Anticorps suivant la revendication 1, qui est un anticorps monoclonal.

3. Anticorps suivant la revendication 1 ou la revendication 2, qui est une forme réduite et S-bloquée de l'anticorps monoclonal produit par la lignée cellulaire ECACC 87 091 606.

4. Anticorps suivant l'une quelconque des revendications 1, 2 ou 3, où les radicaux S-bloquants sont des radicaux alcoyle ou acyle facultativement substitués.

5. Anticorps suivant la revendication 4, où le radical S-bloquant est -CH$_2$CONH$_2$.

6. Réactif anti-Rh(D) comprenant un anticorps monoclonal suivant la revendication 2, en combinaison avec un ou plusieurs autres anticorps anti-Rh(D) ayant une ou plusieurs spécificités de liaison supplémentaires.

7. Réactif anti-Rh(D) suivant la revendication 6, comprenant un anticorps monoclonal qui reconnaît tous les variants Rh(D) sauf D$^{VI}$ et un anticorps monoclonal IgG1 non réduit avec une activité anti-D$^{VI}$.

8. Réactif suivant la revendication 7, où l'anticorps monoclonal IgG1 est celui produit par la lignée cellulaire ECACC 87 091 603.

9. Procédé de détermination de Rh, où un anticorps suivant la revendication 1 est utilisé dans un test d'agglutination direct.

10. Procédé de détermination de Rh, où le réactif suivant la revendication 7 est utilisé dans un test d'agglutination direct et des échantillons négatifs sont ensuite déterminés par un test antiglobuline par lequel les variants D$^u$ et D$^{VI}$ sont détectés.

**11.** Procédé de détermination de Rh suivant la revendication 10, où les globules rouges subissent un lavage unique après le test d'agglutination direct, avant le test antiglobuline.

**12.** Composition pharmaceutique à utiliser en immunisation passive pour prévenir la maladie hémolytique du nouveau-né comprenant au moins un anticorps suivant la revendication 1 ou la revendication 2 en association avec un excipient ou diluant physiologiquement acceptable.

**13.** Procédé de préparation de l'anticorps suivant la revendication 1, comprenant la réduction des liens disulfure de celui-ci en radicaux sulfhydryle et le S-blocage des radicaux sulfhydryle ainsi formés.

**14.** Procédé suivant la revendication 13, dans lequel l'agent réducteur est soluble dans l'eau.

**15.** Procédé suivant la revendication 13, dans lequel le radical S-bloquant est un radical alcoyle ou acyle facultativement substitué.

## FIG . 1